(19) Europäisches Patentamt
European Patent Office
Office européen des brevets

(11) Publication number: 0 214 616
A2

(12) # EUROPEAN PATENT APPLICATION

(21) Application number: 86112224.0

(22) Date of filing: 04.09.86

(51) Int. Cl.⁴: C 07 C 177/00
A 61 K 31/557

(30) Priority: 06.09.85 US 773393

(43) Date of publication of application: 18.03.87 Bulletin 87/12

(84) Designated Contracting States: DE FR GB IT

(71) Applicant: G.D. Searle & Co.
P.O. Box 1045
Skokie Illinois 60076(US)

(72) Inventor: Weier, Richard Mathias
240 Hickory Court
Lake Bluff, Il. 60044(US)

(74) Representative: Wolff, Hans Joachim, Dr.jur. Dipl.-Chem. et al,
Beil, Wolff & Beil Rechtsanwälte Postfach 80 01 40
Adelonstrasse 58
D-6230 Frankfurt am Main 80(DE)

(54) **Acetylenic prostaglandins.**

(57) This invention relates to novel acetylenic prostaglandin derivatives of the following structural formula:

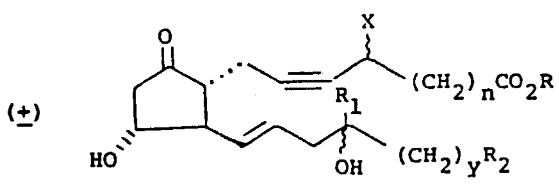

XX

wherein R is hydrogen; or straight or branched chain alkyl of 1 to 6 carbon atoms; $R_1$ is hydrogen; or straight or branched chain alkyl of 1 to 6 carbon atoms; or vinyl ($-CH=CH_2$); $R_2$ is straight or branched chain alkyl of 1 to 6 carbon atoms; or a cycloalkyl group containing 3 to 6 carbon atoms; or phenyl; or phenoxy; n is an integer of from 1 to 4; X is hydrogen; or chloro, bromo, or fluoro; y is an integer of from 1 to 3; ± refers to the compound shown, its mirror image and the mixture of various racemates. These compounds are useful by reason of their gastric antisecretory and cytoprotective activity.

EP 0 214 616 A2

Our No. 25 208

G.D. Searle & Co.

P.O.Box 1045

USA - Skokie, Il. 60076

# ACETYLENIC PROSTAGLANDINS

## Background of the Invention

The present invention relates to novel prostaglandins possessing an acetylenic moiety in the upper side chain in contrast to the olefinic moiety of the $PGE_2$ type prostaglandins. In particular this invention relates to acetylenic prostaglandins of formula XX.

The novel compounds of the present invention display valuable pharmacological properties as is exemplified by their ability to inhibit the gastric secretion stimulated by secretogogues such as histamine and pentagastrin. In addition, these compounds possess the remarkable ability to protect the gastric and intestinal mucosa against the damaging effects of such agents as ethanol and aspirin. This effect has been termed "cytoprotection" (see A.

Robert et al., Gastroenterology, 77, 433 (1979)). Furthermore, these compounds have the surprising advantage of substantially decreased undesirable side effects such as diarrhea and uterine stimulating activity displayed by related substances. The gastric antisecretory activity is determined by standard laboratory means.

Gastric antisecretory agents may be used to treat such diseases as hypersecretion of gastric acid and peptic ulcer. A number of methods to control these conditions exist including gastric antacids, antimuscarinic drugs, $H_2$-receptor blockers and prostaglandins (PG). Goodman and Gilman, Sixth Ed., 1980, pgs. 997, 632, 995-997 and 678.

PG analogs are all known to cause side effects, notably diarrhea. However, the capacity to suppress gastric secretion by these compounds is well documented.

Prior Art

United States Patent 3,974,200 discloses acetylenic prostaglandin, 5,6-dehydro-$PGE_2$ and its analogs useful to reduce and control excessive gastric secretion. The prior art compounds differ from the compounds of this

invention in that they are 15 substituted while the instant compounds are 16 substituted.

Literature reference titled "The Synthesis of 5,6-Acetylenic Prostaglandins" by C. H. Lin, S. J. Stein, and J. E. Pike teaches the synthesis of 5,6-acetylenic analogs of $PGF_2\alpha$ as well as the conversion of said compounds to the E series.

Literature reference titled "Synthesis and Gastric Antisecretory Properties of 4,5 Unsaturated Derivatives of 15-Deoxy-16-Hydroxy-16-Methyl Prostaglandin $E_1$" discloses a compound of the following formula:

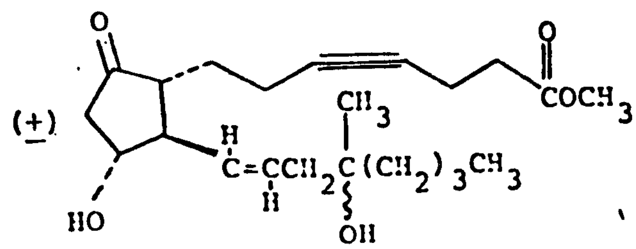

However, the article states that said compound is devoid of gastric antisecretory activity at the highest dose tested (100µg/kg).

Summary of the Invention

The present invention provides for compounds according to the following formula:

(±) 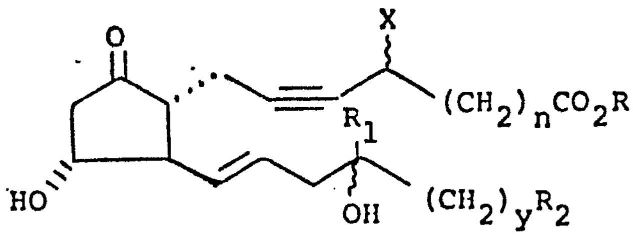 XX

wherein R is

hydrogen; or

straight or branched chain alkyl of 1 to 6 carbon atoms, inclusive.

wherein $R_1$ is

hydrogen; or straight or branched chain alkyl of 1 to 6 carbon atoms, inclusive; or vinyl ($-CH=CH_2$).

wherein $R_2$ is

straight or branched chain alkyl of 1 to 6 carbon atoms, inclusive; or cycloalkyl group containing 3 to 6 carbon atoms; or phenyl; or phenoxy.

wherein n is an integer of from 1 to 4, inclusive.

wherein X is

hydrogen; or chloro, bromo, or fluoro

wherein y is an integer of from 1 to 3, inclusive.

The (±) refers to the compounds shown, its mirror image and the mixture of racemates. Also included in the invention are the individual stereoisomers, and the mixture of isomers, wherein an alpha and beta isomer mixture is represented by the wavy lines in the above formula.

Further, alpha configurations are represented by a dashed line, and beta configurations are represented by a thick line in the above formula.

The alkyl radicals represented in the foregoing structural formula are typified by methyl, ethyl, propyl, butyl, pentyl, and heptyl and the branched-chain radicals thereof.

The cycloalkyl groups represented in the foregoing structural formula are typified by cyclopropyl, cyclobutyl and cyclopentyl.

Preferred embodiments of the present invention are compounds of the following formula:

(±) 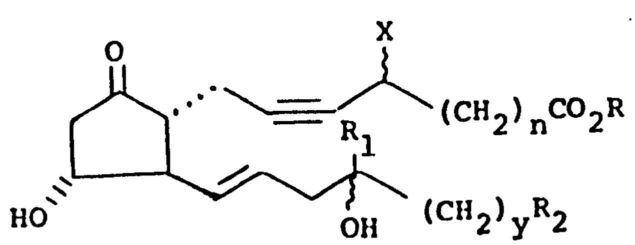 XX

wherein R is
hydrogen; or
methyl

wherein $R_1$ is
methyl

wherein $R_2$ is
straight or branched chain alkyl of 1 to 3 carbon atoms, inclusive; or cycloalkyl group containing 3 to 5 carbon atoms.

wherein n is the integer 2 or 3.

wherein X is
hydrogen; or chloro, bromo, or fluoro.

wherein Y is the integer 2.

The $(\pm)$ refers to the compounds shown, its mirror image and the mixture of racemates. Also included in the invention are the individual stereoisomers, and the mixture of isomers, wherein an alpha and beta isomer mixture is represented by the wavy lines in the above formula.

Further, alpha configurations are represented by a dashed line, and beta configurations are represented by a thick line in the above formula.

The specific assays used to detect gastric antisecretory activity are described as follows:

Heidenhain Pouch Dog Model

Adult female beagle dogs weighing 13-20 kg are prepared with denervated fundic Heidenhain pouches. After a recovery period of at least 4 weeks following surgery, the animals are fasted for approximately 20 hours, then are placed in Pavlov stands and infused intravenously with saline solution. The pouched secretions are collected every 15 minutes and measured for volume and total acidity by titration with 0.1N sodium hydroxide to pH 7.0. Following a 30 minute basal secretion the dogs are infused with a saline solution of histamine dihydrochloride at a dose of 1.0 mg/hr. The volume of the infusion is kept at approximately 13 ml/hr. A steady state plateau of gastric secretion is obtained approximately 1 hour following the start of histamine infusion, at the end of which time the test compound dissolved in an ethanolic iso-osmotic phosphate buffer solution is administered by a single intravenous injection. The duration of the anti-secretory effects is determined and the side-effects, if any, recorded. The compound is rated active if statistically

significant inhibition of secretory parameters occur following compound treatment.

Gastric Fistula Dog Model

Adult female beagle dogs weighing 6-11 kg. are prepared with whole stomach simple Thomas-type gastric cannulas.

Following full recovery from the surgical implantation of the gastric cannula, the dogs are trained to stand quietly, though fully conscious, in Pavlov-type dog restraining slings and are accustomed to intravenous histamine infusion.

Experiments are initiated by depriving dogs of food, but not water, for 18 hours. With an initial infusion of 0.15M sodium chloride, at a constant rate of 6.5 ml/hr, gastric secretions collected in plastic bottles affixed to the cannula, are taken at 15 minute intervals and measured for volume to the nearest 0.1 ml. Following a 30-45 minute basal secretion period, the collection bottles are removed, dosing plugs inserted, and compound administered. A 3.0 ml saline wash follows immediately.

After the end of a 30 minute drug absorption period the stomachs are emptied, collection bottles again attached, and the collections resumed at 30 minute intervals. Simultaneously, the saline infusion is replaced with a

continuous intravenous infusion of histamine dihydrochloride in saline at 15 ug/kg/hr for four hours. Gastric samples are analysed for pH and titratable acidity determinations.

An analysis of the data for each measured or derived variable compares observations recorded following treatment with variables obtained for the same group of animals receiving histamine stimulation alone. Three parameters, gastric juice volume (ml/30 min), acid concentration (mEq/L), and total acid output (mEq/30 min) are analyzed individually. The data thus obtained are analyzed using interval-by-interval paired Student's t-test or two-way analysis of variance to achieve an indication of potency and duration of action. Percentage inhibition is calculated using pooled mean values for the four hour treatment period. Duration of activity is defined as the length of time of significant inhibition.

Meal Stimulated Pavlov-pouch Beagles

To evaluate the gastric antisecretory activity of these prostaglandins, each compound was administered either orally, intravenously or directly into the gastric pouch of surgically-prepared, meal-stimulated Pavlov pouch beagles. A neat solution of each agent was prepared from a stock ethanol solution immediately prior to dosing and

was administered in a vehicle of 20% ethanol and 80% phosphate buffer.

Adult female beagles, 6-11 kg body weight, obtained from either Laboratory Research Enterprises (Kalamazoo, MI) or Hazelton Research Animals (Cumberland, VA) were surgically prepared by implanting a Thomas-type gastric cannula into an innervated (Pavlov) gastric pouch. In all studies gastric secretions were collected from the innervated pouches by gravity drainage. Following recovery each dog was trained to stand quietly in a dog restraining sling and ingest 10-12 ounces of either all beef dog food or cooked ground beef. In the course of these studies, no dog was used more than once a week.

Dogs were deprived of food, but not water, for 24 hours prior to experimentation. Following a 30 minute basal secretion period, the test agent was administered either orally in gelatin capsules, intravenously or directly into the gastric pouch. At the end of an additional 30 minute period to allow for drug absorption, the gastric pouch was emptied of its contents. At this point gastric secretory stimulation was achieved by feeding. The volume (ml/30 min) and titratable acidity (mEq/L) were measured and total acid output (mEq/30 min) was calculated for gastric secretions collected over 30 minute intervals for 240 minutes after compound administration.

The analysis of data was based upon a comparison of treated vs. control experiments. Percentage inhibition for the four hour period was calculated for individual dogs but is expressed as the mean value for those dogs of the control and treated group.

The specific assay used to detect cytoprotective activity is described as follows:

Male, Charles River rats, weighing 180 to 220g, which are food deprived for 24 hours are administered a test compound. Thirty minutes later, each rat is given 1.0 ml of absolute ethanol intragastrically. The rats are sacrificed sixty minutes after alcohol administration and gastric mucosae are visually examined for the presence of lesions. Objective scoring is based on the presence or absence of lesions and data recorded as the number of rats per group completely protected from lesion formation.

Diarrhea is an undesirable side effect commonly associated with antisecretory and cytoprotective prostaglandins. Diarrheogenic activity is demonstrated by the following standardized test. Groups of six adult male Charles River rats, weight range 180 to 200 grams, are fasted for 24 hours prior to administering the test substance. The prostaglandin to be tested is administered intragastrically in iso-osmotic phosphate buffer at a volume of 10 ml/kg at doses ranging from 100 to 3000

microgram/kg. Control animals receive only the vehicle. The rats are placed in individual wire mesh cages and the trays lined with brown paper. Diarrhea is assessed at hourly intervals on an all or none basis for up to eight hours after administration of the prostaglandin. Diarrhea is defined as any loose or watery stool. $ED_{50}$ values are assessed for each hourly diarrheogenic response.

Compounds of this invention were tested as above and found to be antisecretory and cytoprotective. By virtue of these activities, the compounds of the invention are useful in treating and alleviating gastric ulcers in mammals.

The compounds of the present invention are formulated into pharmaceutically acceptable dosage forms by conventional methods known to the pharmaceutical art. For example, the compounds can be administered in oral unit dosage forms such as tablets, capsules, pills, powders, or granules. They also may be administered rectally or vaginally in such forms as suppositories or bougies; they may also be introduced in the form of eye drops, intraperitoneally, subcutaneously, or intramuscularly, using forms known to the pharmaceutical art. In general the preferred form of administration is oral.

An effective but non-toxic quantity of the compound is employed in treatment. The dosage regimen for preventing

or treating symptoms by the compounds of this invention is selected in accordance with a variety of factors including the type, age, weight, sex, and medical condition of the mammal, the severity of the symptoms, the route of administration and the particular compound employed. An ordinarily skilled physician or veterinarian will readily determine and prescribe the effective amount of the agent to prevent or arrest the progress of the condition. In so proceeding, the physician or veterinarian could employ relatively low dosages at first subsequently increasing the dose until a maximum response is obtained.

Initial dosages of the compounds of the invention are ordinarily in the area of 0.25 μg/kg up to at least 50 μg/kg orally. When other forms of administration are employed equivalent doses are administered.

The compounds of this invention are prepared by the general methods illustrated in the accompanying Charts A, B, C and D. Chart A: Furfural, Formula I, reacts with propynyl magnesium halides, II, Grignard reagents prepared by methods known to those skilled in the art from omega-halopropynes, to form the intermediate compounds of Formula III. Preferred reaction conditions include addition at about 0° of a tetrahydrofuran solution of compound I to a diethyl ether solution of the freshly prepared Grignard reagent. Compounds III are typically purified by distillation at reduced pressure. The

intermediates III rearrange upon heating under acidic conditions to form cyclopentenyl compounds of Formula IV.. Preferred conditions include heating at ca. 80-85° in aqueous dioxane containing p-toluenesulfonic acid. Crude compounds IV are typically purified by extraction and column chromatography on silica gel. Compound IV further rearranges under acidic or basic conditions to form the isomeric compounds of Formula V. Preferred conditions include treatment of compounds IV with basic Grade III alumina at room temperature. Protection of alcohol intermediates V, using a protecting group ($R_{10}$) such as trialkylsilyl or tetrahydropyranyl, by reaction in an inert organic solvent affords the protected derivatives, Formula VI. Preferred reagents and conditions include triethylsilyl chloride and imidazole in dimethylformamide at room temperature. The intermediate compounds VI are typically purified by column chromatography on silica gel.

Chart B: Compounds VI react first with organocopper reagents, Formula VII (prepared by the general method described in US Patent 4,271,314) and then with suitable silylating reagent to form intermediates of Formula VIII. Preferred conditions include reaction of VI and VII at ca. -60° in diethyl ether, followed by addition of a trialkylsilyl chloride, preferably with at least one bulky alkyl group (e.g. t-butyldimethylsilyl chloride) which will increase yields and stability, and hexamethylphosphoric triamide, with warming to ca. -20°.

After extracting into an organic solvent, such as diethyl ether, and stripping volatiles, the crude intermediates VIII are typically purified by column chromatography on silica gel. The acetylenic intermediates VIII, are reacted with strong non-aqueous base in an inert organic solvent solution and the appropriate ester aldehyde to provide the intermediates of formula IX. Preferred reaction conditions include reaction of VIII with n-butyllithium in tetrahydrofuran at ca. -30°, followed by the addition of the ester aldehyde at -60° in tetrahydrofuran. Intermediates IX are typically purified by column chromatography on silica gel. Treatment of the intermediates of formula IX with methanesulfonyl chloride gave the compounds of formula X. Reaction conditions include reaction of IX with methanesulfonyl chloride in pyridine at 0°. Treatment of X with sodium iodide, lithium iodide or lithium bromide gave the compounds of formula XI. Dehalogenation of the compounds of formula XI was by treatment of XI with tri-n-butylstannane in benzene. Hydrolysis of the protected compounds of XII under acidic conditions afforded the compounds of Formula XIII. Preferred hydrolytic conditions include a 3:1:1 mixture of acetic acid/tetrahydrofuran/water stirred at room temperature.

Chart C

Compounds IX react with N-(2-chloro-1,1, 2-trifluoroethyl) diethylamine to form intermediates of Formula XV.

Preferred conditions include reaction of IX in anhydrous methylene chloride under a nitrogen atmosphere, followed by the addition of N-(2-chloro-1,1,2- trifluoroethyl) diethylamine. The reaction mixture was stirred at 0° until thin layer chromatography indicated completion of the reaction. After extracting into an organic solvent, such as diethylether, and stripping volatiles, the intermediates are typically purified by column chromatography on silica gel. Hydrolysis of the protected compounds of XV under acidic conditions afforded the compounds of formula XVI. The preferred conditions for hydrolysis are identical to the conditions described in Chart B.

Chart D

Compounds IX react with carbon tetrachloride/hexamethyl phosphorous triamide to form the intermediate compounds XVIII wherein $X_2$ is chloride. Preferred conditions include reaction of IX with carbon tetrachloride in anhydrous ethyl ether at 0° under nitrogen. The resultant solution is then treated with hexamethylphosphorous triamide in anhydrous ethyl ether and stirred for an additional period of time at 0°. After extracting into an organic solvent, such as diethyl ether, and stripping volatiles, the intermediates are typically purified by column chromatography on silica gel.

Compounds IX can react with carbon tetrabromide/triethylamine/hexamethylphosphorous triamide to form the intermediate compounds XVIII wherein $X_2$ is bromide. Preferred conditions include reaction of IX with triethylamine and carbon tetrabromide in ethyl ether containing lithium bromide at -40°. The resultant solution is then treated with hexamethyl phosphorous triamide in ethyl ether and stirred for an additional period of time at -40°. The work-up of the reaction to obtain the compounds of XVIII wherein $X_2$ is bromide is identical to the work-up described for the compounds of XVIII wherein $X_2$ is chloride after the hexamethyl phosphorous triamide addition step.

Compounds of formula XVIII wherein $X_2$ is iodo are prepared first by reacting compounds of formula IX with methane sulfonyl chloride to form the mesylate compound of formula X Chart B. The mesylate compound is then treated with sodium iodide to give the compounds of formula XVIII wherein $X_2$ is iodo. Preferred conditions for the preparation of the mesylate compound are described in Chart B. Preferred conditions for the preparation of the iodo compound include reaction of the mesylate compound with sodium iodide in acetone. The resultant solution is refluxed under a nitrogen atmosphere until thin layer chromatography indicates the reaction to be complete. The reaction mixture is concentrated, redissolved in water and washed with ether. The organic layers are combined,

washed and the volatiles are stripped to leave an oil. The oil is purified by column chromatography.

The intermediates of formula XVIII are deprotected by the method described in Chart B.

A procedure for the preparation of the compounds wherein R in formula XX is hydrogen is described in U.S. Patent 4,271,314.

The following examples further illustrate details for the preparation of the compounds of this invention. The invention, which is set forth in the foregoing disclosure, is not to be construed or limited either in spirit or in scope by these examples. Those skilled in the art will readily understand that known variations of the conditions or processes of the following preparative procedures can be used to prepare these compounds. All temperatures are degree Celsius unless otherwise noted.

CHART A

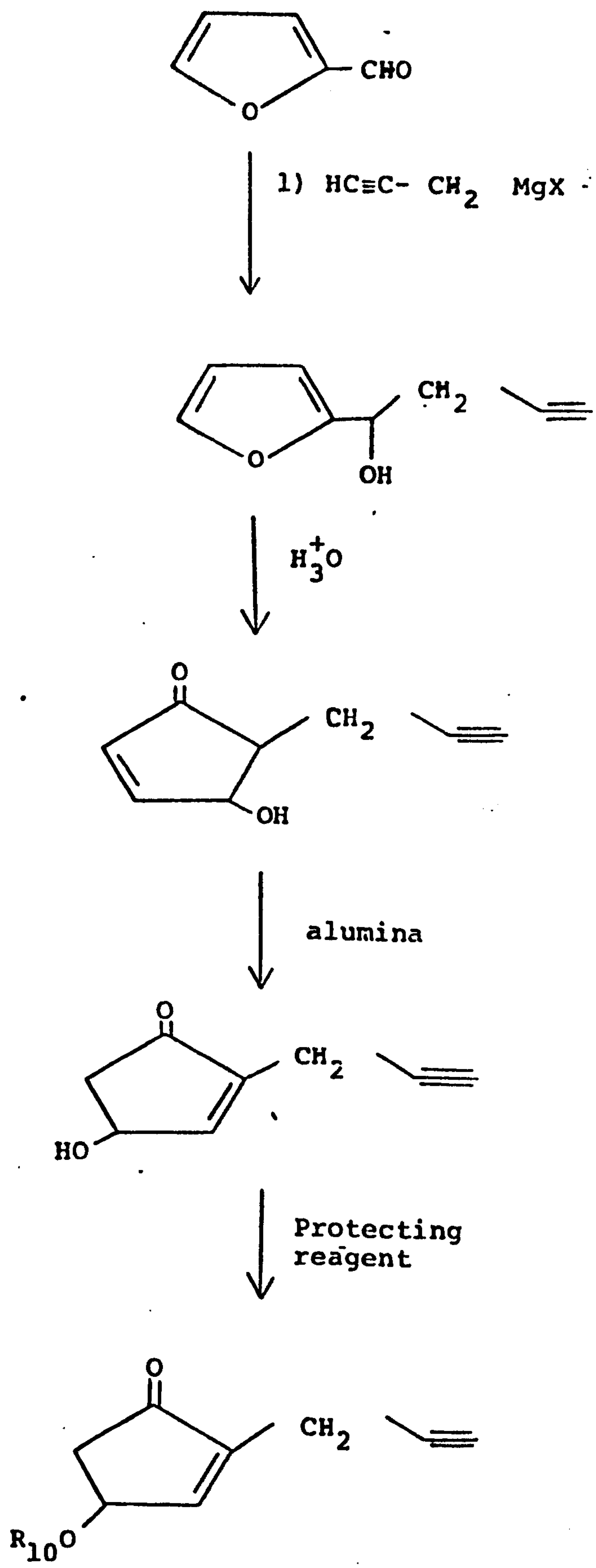


I

II

III

IV

V

VI

CHART B

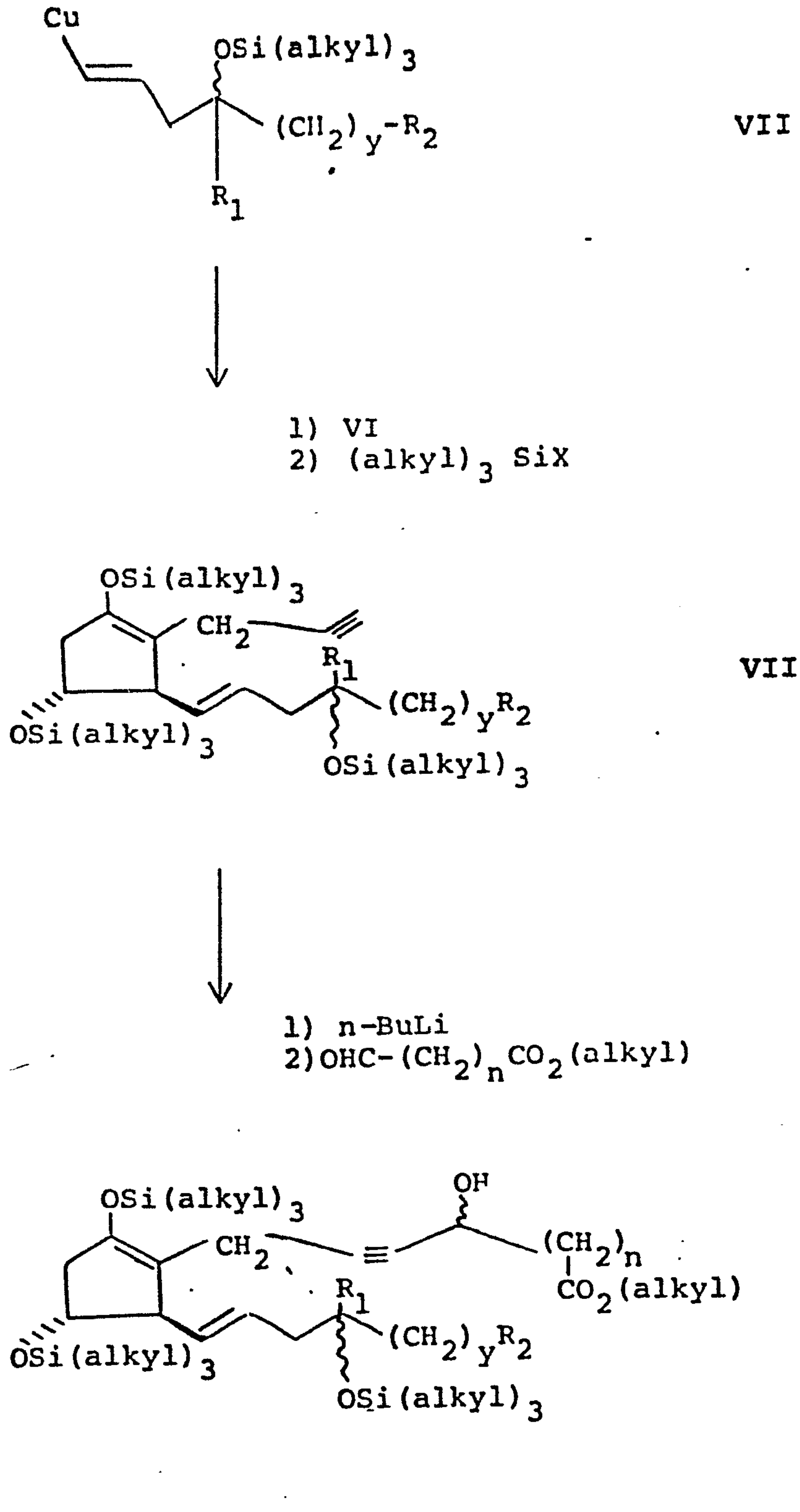


VII

VIII

IX

CHART B (cont.)

Methanesulfonyl chloride

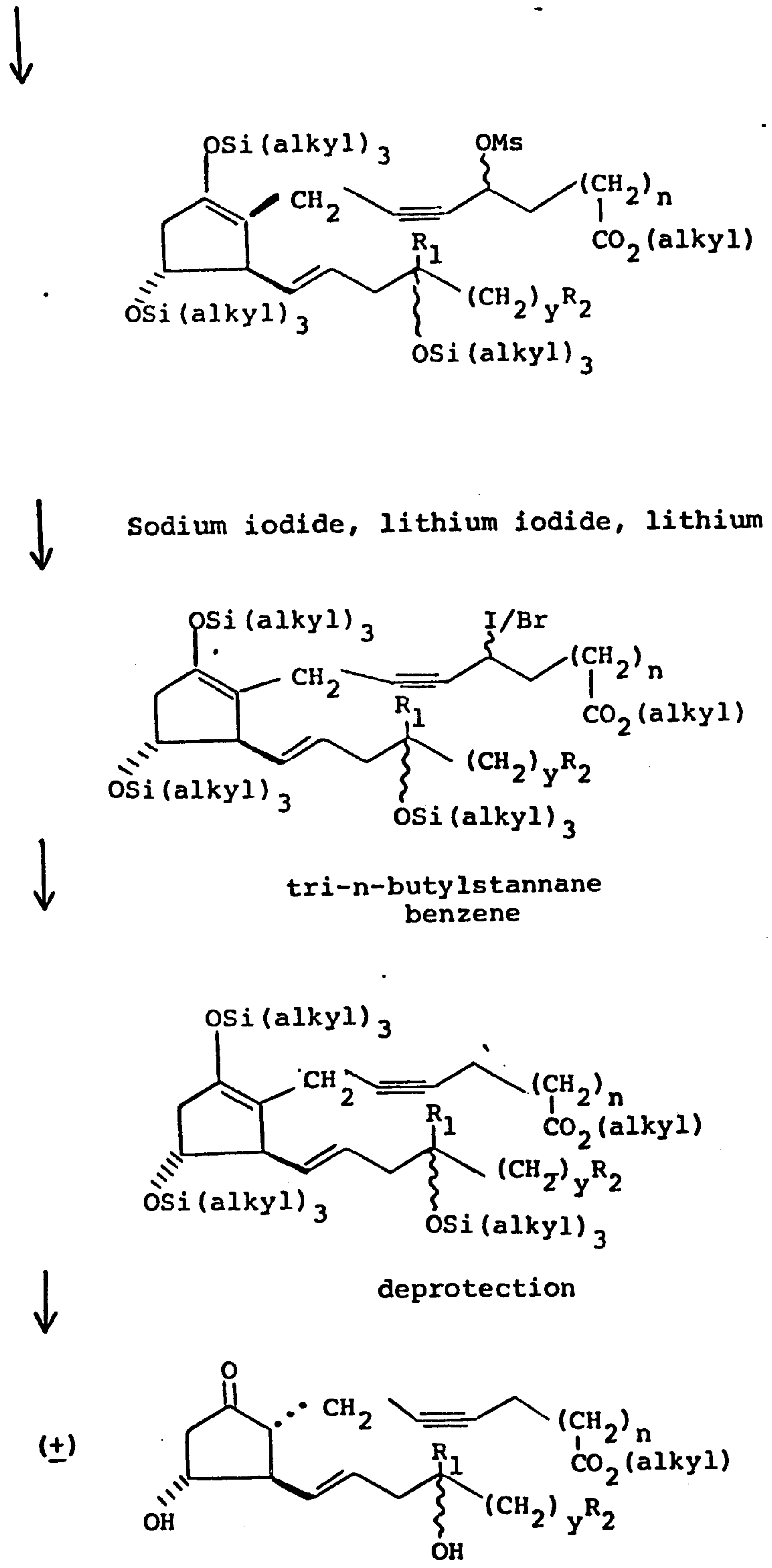


X

XI

XII

XIII

CHART C

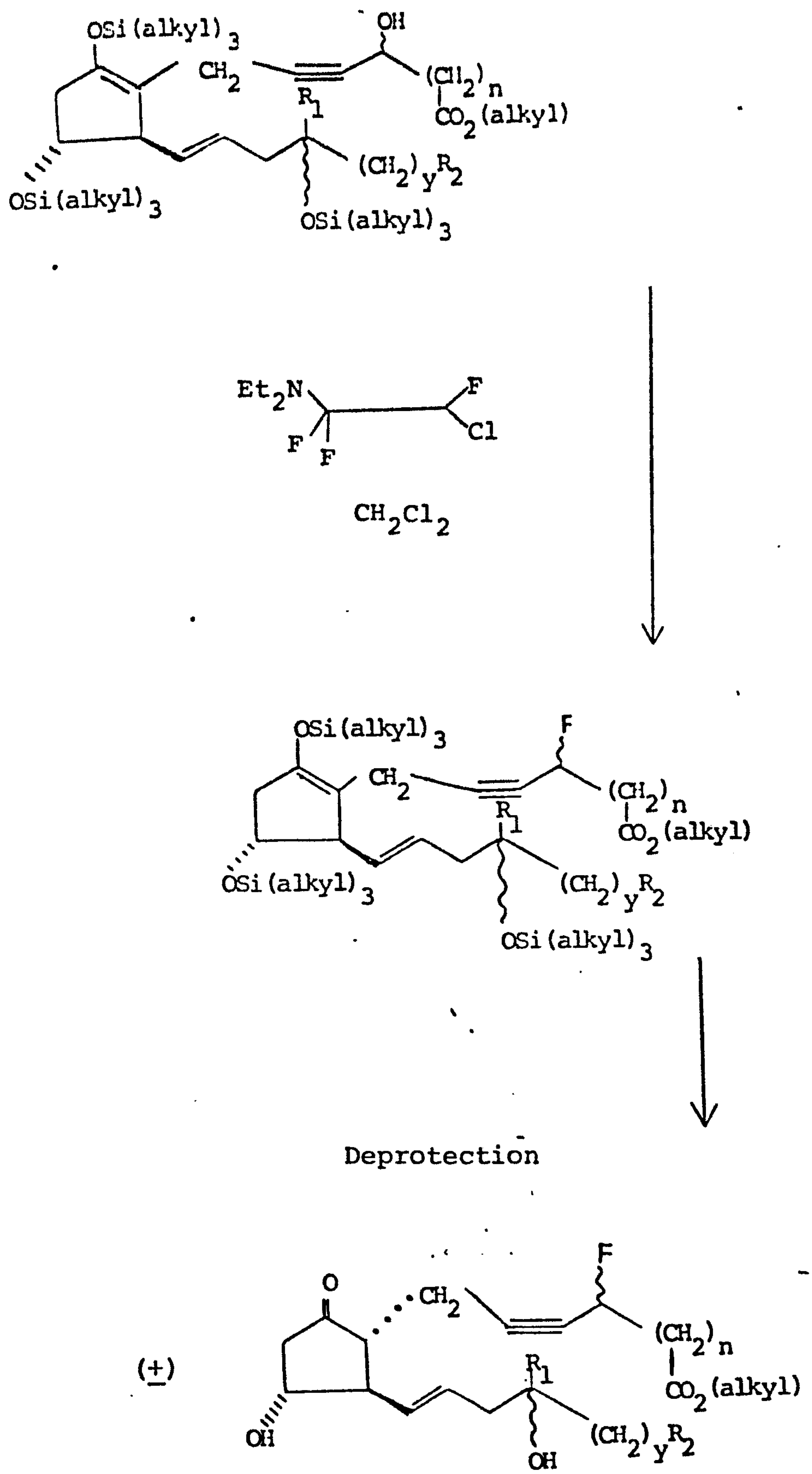


IX

XV

XVI

CHART D

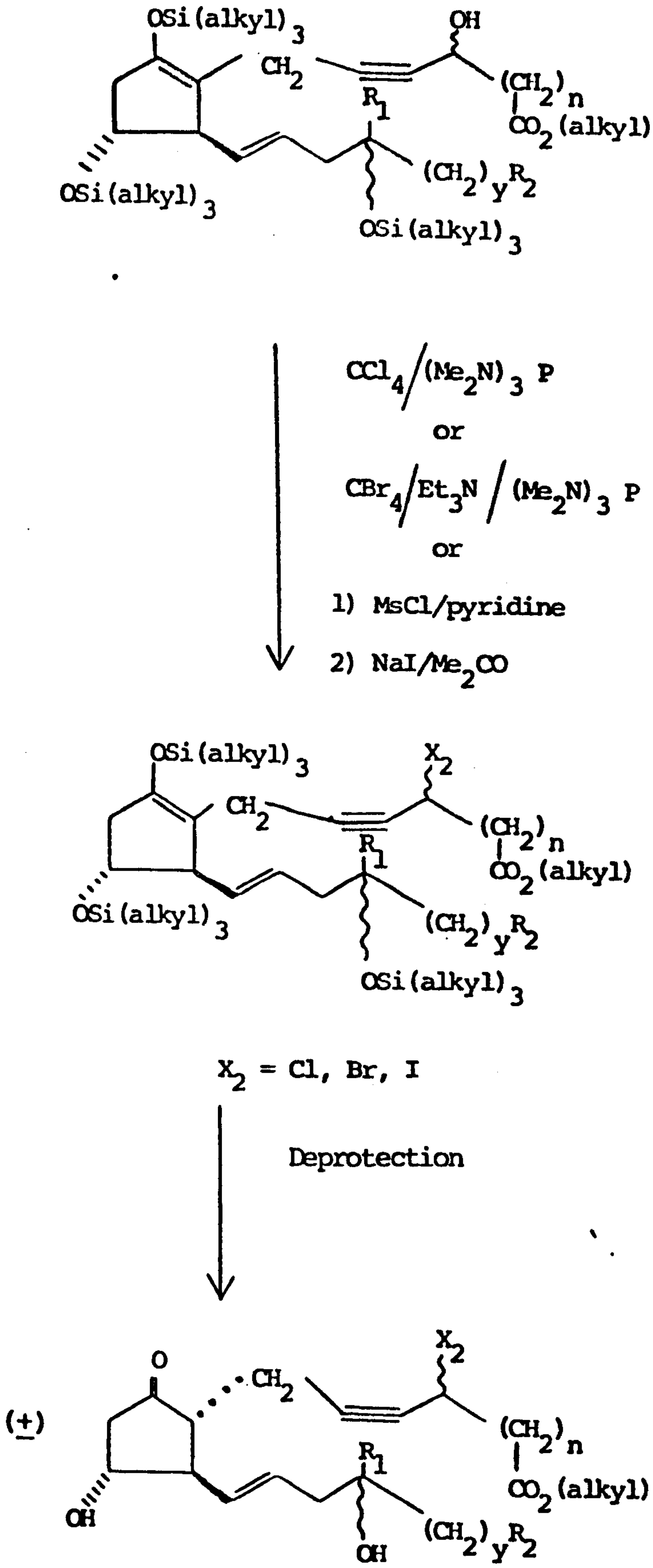


IX

XVIII

XIX

Description of the Preparation of Starting Materials

Example A

Preparation of α-(2)propynyl)-2-furanmethanol

Propargyl magnesium bromide was prepared by adding a solution of propargyl bromide (as 145.5 g of 80%, by weight, solution in toluene; 0.976 mole) in 150 ml of diethyl ether to a slurry of 26 g (1.07 mole) of iodine-activated magnesium and 340 mg of mercuric chloride in 450 ml of ether. The rate of addition was adjusted to maintain a vigorous reflux. After addition was complete, the reaction mixture was stirred at room temperature for one hour and then cooled to 0°. A solution of 75 g (0.78 mole) of 2-furancarboxaldehyde in 400 ml of tetrahydrofuran was added dropwise, and the reaction mixture was stirred at room temperature for fifteen minutes, then poured onto a cold saturated ammonium chloride solution and stirred vigorously. The layers were separated and the aqueous layer was extracted with ether. The organic phase was washed with saturated ammonium chloride solution and with brine, dried over sodium sulfate, filtered, and concentrated to dryness. Distillation of the crude material at 1.0 torr gave 100.6 g of the title compound, b.p.68-72°. Structure assignment was confirmed by the proton nmr spectrum: 2.05 (5, t = 2-3 HZ, ≡ C-H), 2.59 (d of d, J = 2-3 and

5-6 Hz, $-CH_2-C\equiv$), 4.80 (q, J = 5-6 Hz, -C<u>H</u>OH-), 6.27 and 7.32 ppm (furan).

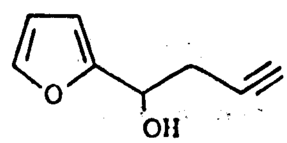

EXAMPLE B

4-hydroxy-2-(2-propynyl)-2-cyclopenten-1-one

To a solution of 40.2 g (0.295 mole) of the title compound of Example 1 in 800 ml of a 8:1 dioxane/water mixture was added 4 g (0.021 mole) of p-toluenesulfonic acid monohydrate. The reaction mixture was heated at 83° for 36 hours under argon, cooled, and diluted with 500 ml of ethyl acetate. The organic phase was washed once with water and two times each with 5% sodium bicarbonate solution and brine solution. The aqueous washes were combined and extracted with ethyl acetate. The combined organic phases were dried over sodium sulfate, filtered, and concentrated. Chromatography of the combined crude materials on silica gel (using 25% ethyl acetate in hexane as eluent) gave 12.45 g of the intermediate compound 4-hydroxy-5-(2-propynyl)-2-cyclopenten-1-one as a viscous

oil. Structure assignment was confirmed by the proton nmr spectrum.

A solution of 14.5 g (0.11 mole) of the cyclopentenone intermediate in 50 ml of ether was poured into a column packed with 282 g of Grade III alumina (6% water by weight). The column was closed and allowed to stand at room temperature for twenty four hours. The product was eluted from the column with mixtures of diethyl ether and ethyl acetate to give 7.3 g of the title compound as a viscous oil. Structure assignment was confirmed by the proton nmr spectrum:

7.5 (mult, = $C\diagdown_{H}$ ), 4.98 (-C$\underline{H}$OH), 2.16 (t, J = 2-3 Hz, ≡ C-$\underline{H}$), 3.07 ppm (mult, J = $-CH_2$ -C≡).

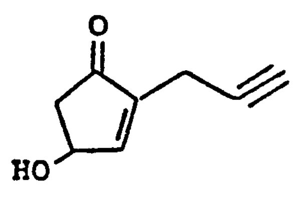

**EXAMPLE C**

2-(2-propynyl)-4-[(triethylsilyl)oxy]-2-cyclopenten-1-one

A solution of 250 mg (1.84 mmole) of the title compound of Example B in 4 ml of dimethylformamide was

treated successively with 200 mg (3 mmole) of imidazole and 300 mg (2 mmole) or triethylsilyl chloride. After stirring for thirty minutes, the reaction mixture was diluted with water, washed with diethyl ether, dried over sodium sulfate, filtered, and concentrated to dryness. The crude material was chromatographed on silica gel to give 0.37 g of the title compound as an oil. Structure assignment was confirmed by the proton nmr spectrum: 2.13 (t, J = 2 Hz, ≡ C-H), 3.08 (mult, $-CH_2-C\equiv$), 4.90 (mult C-11), 7.35 ppm (=C‹H ).

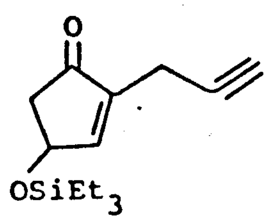

EXAMPLE D

(1,1-dimethylethyl)dimethyl[[3β-[4-methyl-4-[(trimethylsilyl)oxy]-1E-octenyl]-2-(2-propynyl)-4α-[(triethylsilyl)oxy]-1-cyclopenten-1-yl]oxy]silane

A solution of 10 g (0.02 mole) trimethyl[1-methyl-1-[3-(tributylstannyl)-2E-propenyl]pentoxy]silane in 25 ml of tetrahydrofuran was cooled to -60° under an argon atmosphere, and 11.8 ml of a 1.7 M solution of n-butyllithum in hexane (0.02 mole) was added. The

reaction mixture was stirred for forty five minutes, after which a solution of 2.62 g (0.02 mole) of copper-1-pentyne and 6.4 g (0.04 mole) of hexamethylphosphorus triamide in 75 ml of ether was added dropwise. After ten minutes, a solution of 2.4 g (0.01 mole) of the title compound of Example 3 in 20 ml of ether was added, and the reaction mixture was stirred an additional 45 minutes. A solution of 3 g (0.02 mole) of t-butyldimethylsilyl chloride in 15 ml of ether was added, followed by the addition of 25 ml of hexamethylphosphoric triamide. The temperature was allowed to rise to -20°, where it was maintained for one hour. The reaction mixture was poured into 1 N hydrochloric acid and ether. The layers were separated and the organic phase was washed with water, dried over sodium sulfate, filtered, and concentrated to dryness. The crude material was chromatographed on silica gel to give 4 g of the title compound as a viscous oil. Structure assignment was confirmed by the proton nmr spectrum:

1.80 (t, J=1-2Hz, ≡ C-H), 4.95-5.80 (mult, -CH=CH-), 0.90 ppm (t-Bu).

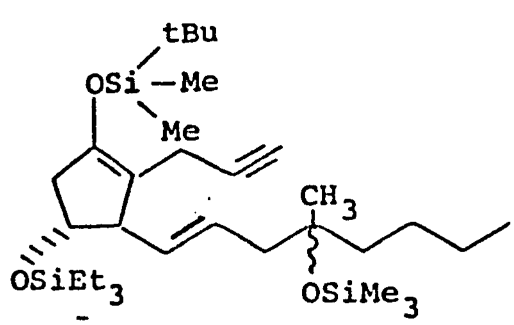

## Example E

Preparation of methyl 4-hydroxy-9-[[(1,1-dimethylethyl) dimethylsily]oxy]-16-methyl-11α-[(triethylsilyl)oxy]-16-[(trimethylsilyl)oxy]prosta-8,13E-dien-5-yn-1-oate

To a stirred solution of 1.993 g (3.45 mmole) of (1,1,-dimethylethyl) dimethyl [[3β-[4-methyl-4-[(trimethylsily) oxy]-1E-octenyl]-2- (2-propynyl)-4α-[(triethylsily)oxy]-1-cyclopenten-1-yl] oxy]silane in 15 ml of anhydrous tetrahydrofuran cooled to -30° under a nitrogen atmosphere, using a trace of triphenyl methyl chloride as an indicator, was added, dropwise, 2.0 ml of 1.66 M n-butyllithium in hexane until the red triphenyl methyl anion endpoint was achieved. After stirring for an additional five minutes, the reaction mixture was cooled to -70° at which time a solution of 1.20 g (10.3 mmole) of methyl 3-formyl propanoate in 2 ml of tetrahydrofuran was added. The resulting reaction mixture was stirred for 90 minutes at -70° before being poured into water. The resulting product was extracted into ether which was then washed with saturated aqueous sodium chloride, filtered, dried over anhydrous sodium sulfate, and concentrated to dryness. The resulting residue was purified by chromatography on a silica gel column, using mixtures of ethyl acetate and hexane as eluent. Removal of the

solvent afforded the title compound having the following structural formula

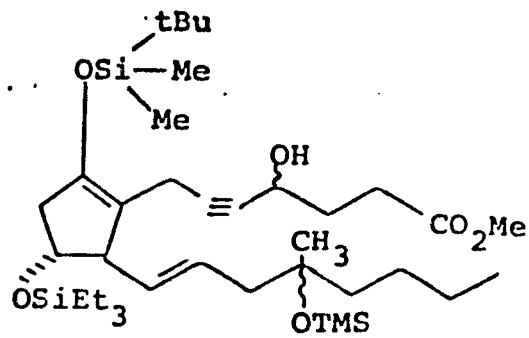

NMR: ($CDCl_3$, ppm) 3.65 (Me), 4.00, (C$\underline{H}$OH), 4.35,

OH

(≡CC$\underline{H}$-), 4.9-5.85 (-C$\underline{H}$=C$\underline{H}$-), 1.15 ($-CH_2-C\underline{H}_3$)

<u>Example F</u>

Preparation of

methyl 5-hydroxy-8-[2-[[(1,1-dimethylethyl) dimethylsilyl]oxy]-5β-[4-methyl-4-[(timethylsilyl)oxy] -1E-octenyl]-4α-[(tiethylsilyl)oxy]-1-cyclopenten-1-yl] -6-octynoate

The title compound was prepared by the method of Example E using 658 mg of (1,1-dimethylethyl)dimethyl[[3β-[4-methyl-4-[(trimethylsilyl)oxy]-1E-octenyl]-2-(2-propynyl)-4α-[(triethylsilyl)oxy]-1-cyclopenten-1-yl]oxy]silane in 8 ml of tetrahydrofuran, using 0.89 ml of 1.53M <u>n</u>-butyllithium in hexane, and 443 mg of

$OHC(CH_2)_3CO_2Me$ in 2 ml of tetrahydrofuran. Chromatography of the crude material on a silica gel column using 20% ethyl acetate/hexane as the eluent afforded the title compound having the following structure

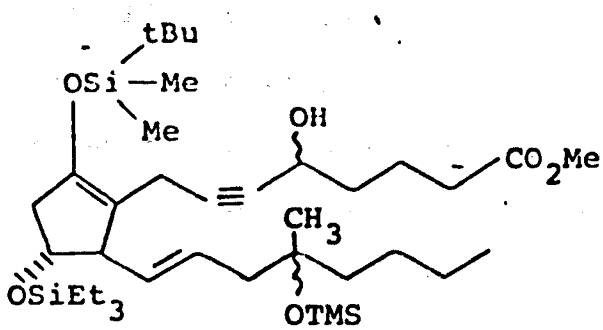

Example G

Preparation of

Methyl 5-chloro-8-[2-[[1,1-dimethylethyl)dimethylsilyl] oxy[-5β-[4-methyl-4-[(trimethylsilyl)oxy]-1E-octenyl]-4α-[(triethylsilyl)oxy]-1-cyclopenten-1-yl]-6-octynoate

A solution of methyl 5-hydroxy-8-[2-[[(1,1-dimethylethyl)-dimethylsilyl]oxy]-5β-[4-methyl-4-[(trimethylsilyl)-oxy]-1E-octenyl]-4α-[(triethylsilyl)oxy]-1-cyclopenten-1-yl]-6-octynoate (258 mg, 0.36 mmol) and carbon tetrachloride (278 mg, 1.80 mmol) in anhydrous ethyl ether (12 ml) was stirred at 0° under nitrogen. To this solution was added hexamethylphosphorous triamide (182 mg, 1.12 mmol) in anhydrous ethyl ether (3 ml). The reaction was stirred for 2 hours at 0°. The reaction was poured

onto saturated aqueous sodium chloride solution and extracted four times with diethyl ether. The combined ether layers were backwashed three times with saturated aqueous sodium chloride solution and dried over sodium sulfate. Filtration of the drying agent and stripping the filtrate gave 282 mg of brown oil. This oil was chromatographed on silica using 2% ethyl acetate/hexane as the eluent to give 221 mg of the pure chloride, title compound having the following structure

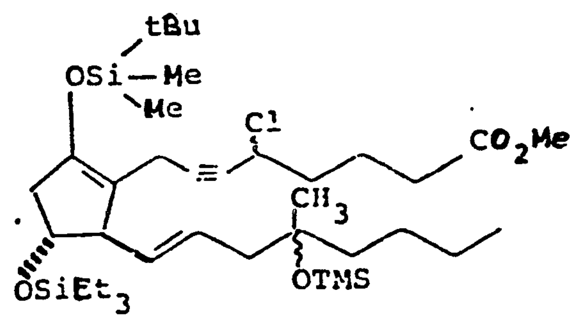

Description of the Preferred Embodiments

Example 1

Methyl 9-[[(1,1-dimethylethyl)dimethylsilyl]oxy]-16-methyl-4-[(methylsulfonyl)oxy]-11α-[(triethylsilyl)oxy]-16-[(trimethylsilyl)oxy]prosta-8,13E-dien-5-yn-1-oate

To a stirred cold (0°) solution of methyl 4-hydroxy-9-[[1,1-dimethylethyl)dimethylsily]oxy]-16-methyl-11α-[(triethylsilyl)oxy[-16[(trimethylsily)oxy]prosta-8-13E-dien-5-yn-1-oate (product of preparation E) (310 mg, 0.454 mmol) in pyridine (5 ml) was added methane sulfonyl chloride (0.15 ml, 222 mg, 1.94 mmol). The reaction mixture was stirred at 0° until thin layer chromatography (silica gel, 2% ethyl acetate/benzene) showed the reaction to be complete. The reaction was poured onto water and extracted three times with ethyl ether. The combined ether layers were washed three times with saturated aqueous sodium chloride solution and dried over sodium sulfate. Filtration of the drying agent and stripping the filtrate gave a crude oil containing white insoluble material. The crude product was redissolved in a small amount of ethyl ether, filtered, stripped and further dried under a nitrogen stream to give 351 mg of the titled product.

NMR ($CDCl_3$, 80 MHz): 3.65 ($-OCH_3$); 3.05 ($CH_3SO_3-$); 3.95 (C-11); 4.97-5.70 ppm (multiplet C-13, 14 and -C<u>H</u> OMs-)

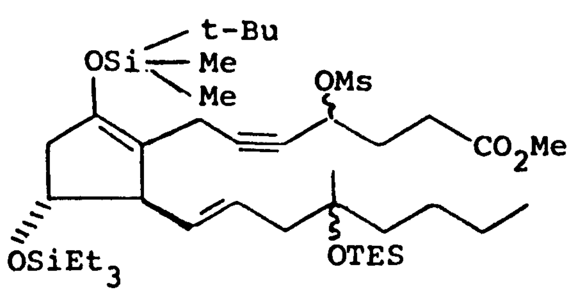

## Example 2

Methyl 4-iodo-9-[[1,1-dimethylethyl)dimethylsily]oxy]-16-methyl-11α-[(triethylsilyl)oxy]-16-[(trimethylsily)oxy] prosta-8-13E-dien-5-yn]-1-oate

A solution of the mesylate of Example 1 (713 mg, 0.91 mmol) and sodium iodide (560 mg, 3.74 mmol) in acetone (15 ml) was refluxed under a nitrogen atmosphere for 75 min. Thin layer chromatography (silica gel, 20% ethyl acetate/hexane) showed no starting material remaining.

The reaction mixture was concentrated on a rotary evaporator and the residue was dissolved in water and washed three times with ethyl ether. The combined ether layers were washed once with saturated aqueous sodium chloride solution, twice with 2% aqueous sodium thiosulfate solution and twice with saturated aqueous sodium chloride solution. After drying over sodium sulfate filtration and stripping the filtrate, the residual oil was flash chromatographed on silica gel using 2% ethyl acetate/hexane to give 536 mg of the titled product.

Nmr ($CDCl_3$, 80 MHz): 3.66 ($-OCH_3$); 3.97 (C-11); 4.60 (-C<u>H</u>I-); 5.30 ppm (multiplet C-13,14)

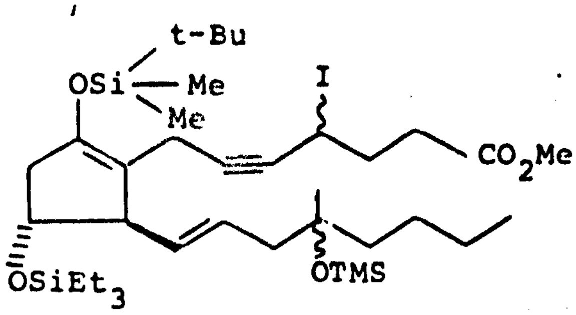

Example 3

Methyl 9-[[1,1-dimethylethyl)dimethylsily]oxy]-16-methyl-11α-[triethylsilyl)oxy]-16-[(trimethylsilyl)oxy]prosta-8-13E-dien-5-yn]-1-oate

A solution of the product of Example 2 (522 mg, 0.66 mmol) and tri-n-butylstannane (576 mg, 1.98 mmol) in benzene (10 ml) containing bisazoisobutyronitrile (22 mg) was refluxed with stirring for one hour under an argon atmosphere. Thin layer chromatography (silica gel, 2% ethyl acetate/benzene) showed the reaction to be complete. The reaction was stripped on the rotary evaporator and further blown dry with a nitrogen stream. The residue was purified by chromatography on silica gel using 100% hexane and 2% ethyl acetate/hexane to give 269 mg of the titled product.

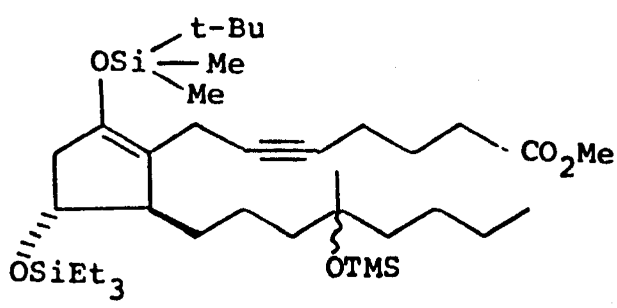

Example 4

Methyl 11α, 16-dihydroxy-16-methyl-9-oxoprost-13E-en-5-yn-1-oate

A solution of the product of Example 3 (87 mg, 0.13 mmol) in glacial acetic acid/tetrahydrofuran/water (3/1/1, 5ml) was stirred at room temperature for 20 hrs. The reaction was poured onto water and extracted four times with ethyl ether. The combined ether layers were washed twice with saturated sodium chloride solution and dried over sodium sulfate. The drying agent was filtered, the filtrate stripped on the rotary evaporator and the residual oil chromatographed on Woelm silica using 55% ethyl acetate/hexane to give 40 mg of the titled product as a colorless oil.

Nmr ($CDCl_3$, 80 $MH_z$): 0.92 (C-20); 1.17 (C-16 $CH_3$); 3.65 ($-OCH_3$); 4.10 (C-11); 5.55 (multiplet C-13,14)

(±) 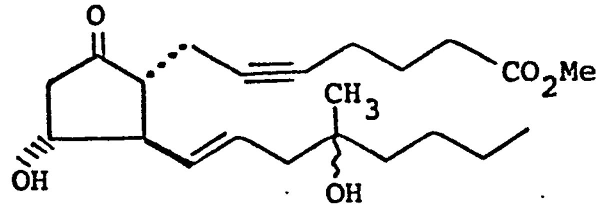

Example 5

(±)Methyl 5-chloro-8-[3α-hydroxy-2β-(4-hydroxy-4-methyl-1E-octenyl)-5-oxo-1α-cyclopentyl]-6-octynoate.

A solution of methyl 5-chloro-8-[2-[[(1,1-dimethylethyl) dimethylsilyl]oxy]-5β-[4-methyl-4-[(trimethylsilyl)oxy]-1E-octenyl]-4α-[triethylsilyl)oxy]-1-cyclopenten-1-yl]-6-octynoate (product of Example G) (188 mg, 0.26 mmol) glacial acetic acid/tetrahydrofuran/water (3/1/1, 15ml) was stirred at room temperature for 20 hrs. The reaction was worked up in the same manner as described in Example 4. The crude product was chromatographed on Woelm silica using 55% ethyl acetate/hexane to give 130 mg of the titled product as a colorless oil.

Nmr ($CDCl_3$, ppm): 4.51 (-C<u>H</u>Cl-); 3.65 ($-OCH_3$); 5.50 (multiplet C-13, 14); 0.90 (C-20); 4.10 (C-11)

(±) 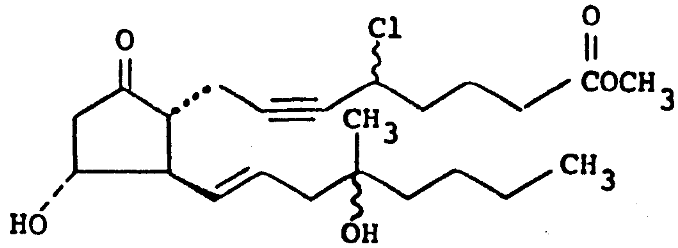

Example 6

Methyl 8-[2[[1,1,dimethylethyl)dimethylsilyl)oxy]-5β-[4-methyl-4-[(trimethylsilyl)oxy]-1E-octenyl]-4α-[triethylsilyl) oxy]-1-cyclopenten-1-yl]-6-octynoate

A solution of methyl 5-chloro-8-[2-[[1,1-dimethylethyl) dimethylsilyl]oxy]-5β-[4-methyl-4-[(trimethylsilyl)oxy]-1E-octenyl]-4α-[(triethylsilyl)oxy]-1-cyclopenten-1-yl]-6-octynoate (239 mg, 0.33 mmol) and tri-n-butylstannane (480 mg, 1.65 mmol) in benzene (7 ml) containing a trace of bisazoisobutyronitrile (5 mg) was refluxed with stirring under an argon atmosphere for 5 hrs. Thin layer chromotography (silica gel, 10% ethyl acetate/hexane) showed only a trace of starting material remaining. Benzene was removed from the reaction solution using a nitrogen stream. The residual yellow oil was chromatographed on Woelm silica using 2% ethyl acetate/hexane as the eluent to give 119 mg of the titled product contaminated with a trace amount of starting material.

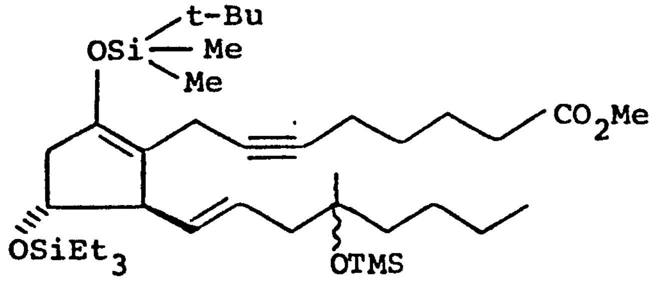

Example 7

(±) Methyl 8-[3α-hydroxy-2β-(4-hydroxy-4-methyl-1E-octenyl)-5-oxo-1α-cyclopentyl]-6-octynoate

A solution of the product of Example 6 (119 mg) in glacial acetic acid/tetrahydrofuran/water (3/1/1, 6.5 ml) was stirred for 20 hrs at room temperature. The reaction mixture was concentrated on the rotary evaporator poured onto water and extracted four times with ethyl ether. The combined ether layers were washed three times with saturated sodium chloride solution and dried over sodium sulfate.

The drying agent was filtered and the filtrate concentrated on the rotary to give a colorless oil. Purification of this oil, by successive chromatographies on silica gel using low pressure, then high pressure techniques gave the titled product as a colorless oil.

(±)

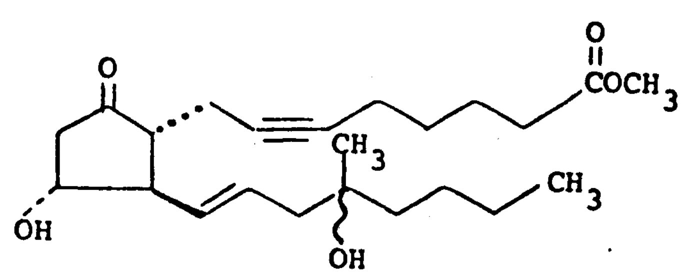

Example 8

Methyl 4-bromo-9-[[(1,1-dimethylethyl) dimethylsily]oxy] -16-methyl-11α-[(triethylsilyl)oxy]-16-[(trimethylsilyl) oxy] prosta-8,13E-dien-5-yn-1-oate.

A solution of methyl 4-hydroxy-9-[[(1,1-dimethylethyl) dimethylsily]oxy]-16-methyl-11α-[(triethylsilyl)oxy]-16-[(trimethylsilyl)oxy] prosta-8,13E-dien-5-yn-1-oate (192 mg, 0.28 mmol), triethylamine (88 mg, 0.87 mmol) and carbon tetrabromide (277 mg, 0.83 mmol) in ethyl ether (7 ml) containing lithium bromide (23 mg, 0.26 mmol) was cooled to -40°. A solution of hexamethylphosphorous triamide (145 mg, 0.89 mmol) in ether (1 ml) was added and the reaction was stirred for 1.25 hr at -40°.

The reaction was poured onto water and extracted four times with ether. The combined ether layers were washed three times with saturated sodium chloride solution and dried over sodium sulfate. The drying agent was filtered, the filtrate was stripped on the rotary evaporator and the residue flash chromotographed on Woelm silica using 2% ethyl acetate/hexane to give 172 mg of the titled product.

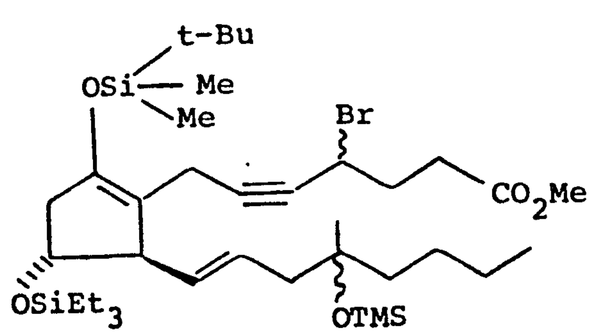

Example 9

(±)Methyl 4-bromo-11α,16-dihydroxy-16-methyl-9-oxo-prost-13E,en-5-yn-1-oate

The titled compound is prepared by the method described in Example 5 using the bromo compound of Example 8 instead of the methyl 5-chloro-8-[2-[[(1,1-dimethyl ethyl) dimethylsilyl]oxy]-5β-[4-methyl-4-[(trimethylsilyl)oxy]-1E-octenyl]-4α-[triethylsilyl)oxy]-1-cyclopenten-1-yl]-6-octynoate.

(±)

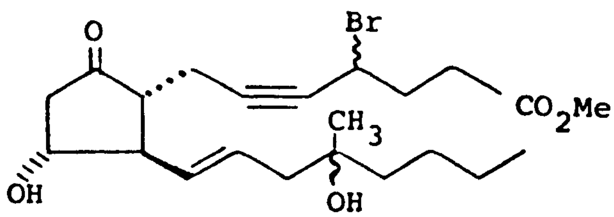

Example 10

Methyl 4-fluoro-9-[[(1,1-dimethylethyl)dimethylsily]oxy]-16-methyl-11α[(triethylsily)oxy]-16-[(trimethylsilyl)oxy] prosta-8,13E-dien-5-yn-1-oate

A solution methyl 4-hydroxy-9[[(1,1-dimethylethyl) dimethylsily)oxy]-16-methyl-11α-[(triethylsilyl)oxy]-16-[(trimethylsilyl) oxy]prosta-8,13E-dien-5-yn-1-oate (354 mg, 0.5 mmol) in anhydrous methylene chloride (9 ml) is stirred at 0° under a nitrogen atmosphere.

N-(2-chloro-1,1,trifluoroethyl) diethylamine (150 mg, 0.79 mmol) is added. The reaction is stirred at 0° until thin layer chromatography shows that the product had formed. The reaction is poured onto ethyl ether and saturated sodium chloride solution. The layers are separated and the aqueous layer is washed twice more with ethyl ether. The combined organic layers are washed twice with saturated sodium chloride solution and dried over sodium sulfate.

The drying agent is filtered and the filtrate stripped in vacuo. The residual oil is chromatographed on silica gel using mixtures of ethyl acetate and hexane as the eluents to give the titled product.

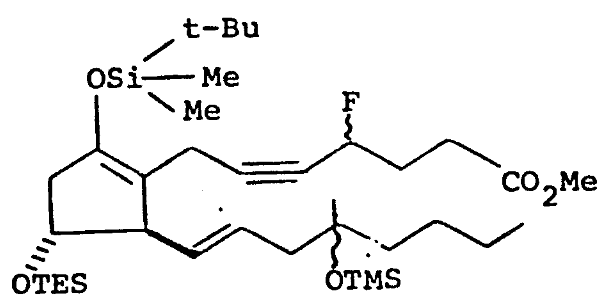

Example 11

(±)Methyl 4-fluoro-11α,16-dihydroxy-16-methyl-9-oxo-prost-13E-en-5-yn-1-oate

The above titled product is prepared using the final product of Example 10 (115 mg) using a 3:1:1 solution of acetic acid/tetrahydrofuran/water (6.5ml) and utilizing the procedure described in Example 5.

(±) 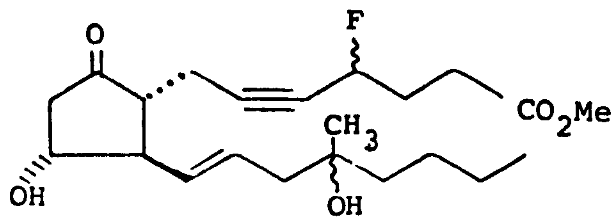

Example 12

Methyl 5-iodo-8-[2[[1,1-dimethylethyl)dimethylsilyl]oxy]-5β-[4-methyl-4-[(trimethylsilyl)oxy]-1E-octenyl]-4α[(triethylsilyl)oxy]-1-cyclopenten-1-yl]-6-octynoate

Lithium iodide (68 mg, 0.51 mmol) was added to a stirred cold (0°) solution of methyl 8-[2-[[(1,1-dimethylethyl)dimethylsilyl]oxy]-5-[4-methyl-4-[(trimethylsilyl)oxy]-1E-octenyl]-4-[(triethylsilyl)oxy]-1-cyclopenten-1-yl]-5-[(methylsulfonyl)oxy]-6-octynoate. (135 mg, 0.17 mmol) and triethylamine (53 mg, 0.52 mmol) in tetrahydrofuran (7 ml). The reaction was stirred at 0° for 15 min, then room temperature for 7 hr. At this time thin layer chromatography (silica gel, 20% ethyl acetate/hexane)

showed the reaction to be complete. The reaction was poured onto water and extracted three times with ethyl ether. The combined ether layers were washed twice with saturated sodium chloride solution and dried over sodium sulfate. The drying agent was filtered, the filtrate stripped on the rotary evaporator and the residue flash chromatographed on Woelm silica using 2% ethyl acetate/hexane to give 77 mg of the titled compound as a colorless oil.

Nmr (ppm): 3.65 ($-OCH_3$); 4.51 (-C <u>H</u>I); 4.00 (C-11); 5.00-5.75 (multiplet C-13,14)

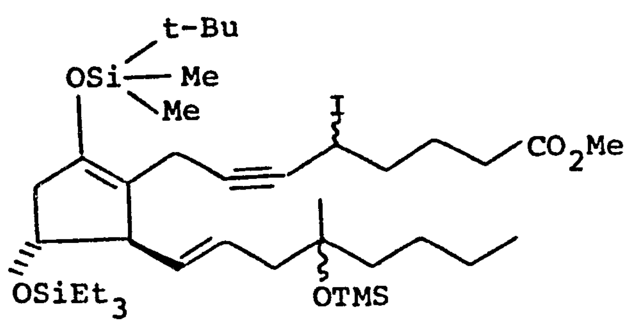

Example 13

(±)Methyl 5-iodo-8-[3α-hydroxy-2β-(4-hydroxy-4-methyl-1E-octenyl)-5-oxo-1α-cyclopentyl]-6-octynoate

The titled compound is prepared by the method described in Example 5 using the iodo compound of Example 12 instead of the methyl 5-chloro-8-[2-[[(1,1-dimethyl ethyl) dimethyl silyl]oxy]-5β-[4-methyl-4-[(trimethylsilyl)oxy]-1E-octenyl]-4α-[triethylsilyl)oxy]-1-cyclopenten-1-yl]-6-octynoate.

(±) 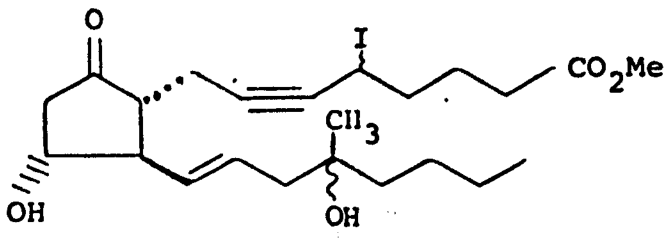

What is claimed is:

1. A compound of the formula

(±) 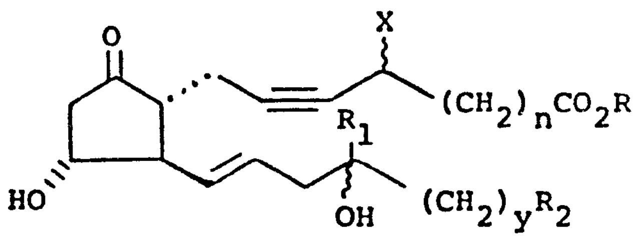 XX

wherein R is

hydrogen; or

straight or branched chain alkyl of 1 to 6 carbon atoms, inclusive

wherein $R_1$ is

hydrogen; or straight or branched chain alkyl of 1 to 6 carbon atoms, inclusive; or vinyl ($-CH=CH_2$)

wherein $R_2$ is

straight or branched chain alkyl of 1 to 6 carbon atoms, inclusive; or cycloalkyl group containing 3 to 6 carbon atoms; or phenyl; or phenoxy

wherein n is an integer of from 1 to 4, inclusive.

wherein X is

hydrogen; or chloro, bromo, fluoro or iodo

wherein y is an integer of from 1 to 3, inclusive

( ± ) refers to the compound shown, its mirror image and the mixture of racemates.

2. A compound according to Claim 1
wherein R is
hydrogen; or
methyl

wherein $R_1$ is
methyl

wherein $R_2$ is
straight or branched chain alkyl of 1 to 3 carbon atoms, inclusive; or cycloalkyl group containing 3 to 5 carbon atoms.

wherein n is the integer 2 or 3.

wherein X is
hydrogen; or chloro, bromo, or fluoro.

wherein Y is the integer 2.

3. ( ± ) Methyl 11α,16-dihydroxy-16-methyl-9-oxoprost -13E-en-5-yn-1-oate, a compound according to Claim 2.

4. ( ± ) Methyl 8-[3α-hydroxy-2β-(4-hydroxy-4-methyl-1E-octenyl)-5-oxo-1α-cyclopentyl]-6-octynoate, a compound according to Claim 2.

5. ( ± ) Methyl 5-chloro-8-[3α-hydroxy-2β-(4-hydroxy-4-methyl-1E-octenyl)-5-oxo-1α-cyclopentyl]-6-octynoate, a compound according to Claim 2.